# EUROPEAN PATENT SPECIFICATION

(11) **EP 4 271 260 B1**
(45) Date of publication and mention of the grant of the patent: **29.01.2025**
(21) Application number: 22715119.8
(22) Date of filing: 23.02.2022
(51) Int. Cl.: A61B 5/022, A61B 5/0225, G16H 50/30, A61B 5/024, A61B 5/00

(54) **AN APPARATUS AND A METHOD FOR MEASURING FLOW RESISTANCES OF BLOOD VESSELS**
VORRICHTUNG UND VERFAHREN ZUR MESSUNG DES STRÖMUNGSWIDERSTANDES VON BLUTGEFÄSSEN
APPAREIL ET PROCÉDÉ DE MESURE DE RÉSISTANCES À L'ÉCOULEMENT DE VAISSEAUX SANGUINS

(30) Priority: 04.03.2021 FI 20215239
(43) Date of publication of application: 08.11.2023
(73) Proprietor: Turun Yliopisto, 20014 Turun yliopisto (FI)
(72) Inventor: KAISTI, Matti, 20720 Turku (FI); PANULA, Tuukka, 20100 Turku (FI); SIRKIÄ, Jukka-Pekka, 20100 Turku (FI)
(74) Representative: Väänänen, Janne Kalervo
(86) International application number: PCT/FI2022/050117
(87) International publication number: WO 2022/184974

(56) References cited:
- WO-A1-2020/144397
- US-A1- 2016 367 154
- GOHICHI TANAKA ET AL: "A novel photoplethysmography technique to derive normalized arterial stiffness as a blood pressure independent measure in the finger vascular bed;Normalized arterial stiffness index in the finger vascular beds", PHYSIOLOGICAL MEASUREMENT, INSTITUTE OF PHYSICS PUBLISHING, BRISTOL, GB, vol. 32, no. 11, 26 October 2011 (2011-10-26), pages 1869 - 1883, XP020213466, ISSN: 0967-3334, DOI: 10.1088/0967-3334/32/11/003
- JING LIU ET AL: "Multi-Wavelength Photoplethysmography Enabling Continuous Blood Pressure Measurement With Compact Wearable Electronics", IEEE TRANSACTIONS ON BIOMEDICAL ENGINEERING, vol. 66, no. 6, 1 June 2019 (2019-06-01), USA, pages 1514 - 1525, XP055657857, ISSN: 0018-9294, DOI: 10.1109/TBME.2018.2874957

## Description

### Field of the disclosure

The disclosure relates to an apparatus and a method for measuring flow resistances of blood vessels. Furthermore, the disclosure relates to a computer program for measuring flow resistances of blood vessels.

### Background

In many cases, quantitative assessment of vascular pathology and its progression involves measurement and estimation of different quantitative measures such as for example: blood pressure, a blood vessel cross sectional area, a blood vessel mean diameter, blood vessel stiffness, and blood vessel flow resistance. For example, abnormal blood vessel stiffness and flow resistance are associated with an increased risk of cardiovascular events such as myocardial infarction and stroke, which are two leading causes of death in the developed world. An increase in arterial stiffness and flow resistance may increase the load of the heart, since the heart needs to perform more work to maintain a required blood flow volume. Over time, this increased workload may cause left ventricular hypertrophy and left ventricular remodelling, which can lead to a heart failure. The increased workload may also be associated with a higher heart rate, a proportionately longer duration of systole, and a reduction of duration of diastole. This decreases the amount of time available for perfusion of cardiac tissue, which mainly occurs during diastole. Thus, a hypertrophic heart, which has a greater oxygen demand, may have a compromised supply of oxygen and nutrients.

Due to the reasons of the kind mentioned above, several techniques have been developed to measure and/or estimate different quantitative measures related to blood vessels. Publication Jukka-Pekka Sirkiä et al.: Multi-Wavelength Photoplethysmography Device for the Measurement of Pulse Transit Time in the Skin Microvasculature, Computing in Cardiology, 2020-09-16 describes a multiwavelength photoplethysmography "MWPPG" device for studying the skin microvasculature. The device utilizes the fact that the penetration depth of light into the skin is depended on the light wavelength. Thus, the device allows to study blood vessels at different depths.

Publication US2017172430 describes a method for cuff-less blood pressure measurement. The method comprises recording a physiological signal and multi-wavelength photoplethysmography "PPG" signals from a predetermined body part, deriving the depth-specific PPG signal reflecting the arterial blood volume with the physiological signal as a reference, calculating the pulse transit time "PTT" from the physiological signal and the derived arterial blood PPG signal, and calculating the blood pressure from the calculated PTT and blood pressure relationship.

Publication US2019336016 describes a device for non-invasive capillary blood pressure measurement. The device comprises a front end in contact with a body to compress and decompress capillaries in tissue, a pressure control module for regulating contact pressure between the front end and the tissue, a pressure transducer coupled to the front end for measuring the contact pressure, a capillary sensing module for detecting capillary pulsations under the contact pressure modulation, and a computing system for running an algorithm to determine capillary pressure based on the capillary pulsations and the contact pressure modulation.

Publication Gohichi Tanaka et al: "A novel photoplethysmography technique to derive normalized arterial stiffness as a blood pressure independent measure in the finger vascular bed; Normalized arterial stiffness index in the finger vascular beds", Physiological Measurement, Institute of Physics Publishing, Bristol, GB, vol. 32, no. 11, 26 October 2011, pages 1869-1883 describes a method where a finger arterial elasticity index "FEI" is defined as a parameter which denotes the curvilinearity of an exponential model of a pressure-volume relationship.

Publication Jing Liu et al.: "Multi-Wavelength Photoplethysmography Enabling Continuous Blood Pressure Measurement with Compact Wearable Electronics", IEEE Transactions on Biomedical Engineering, vol. 66, no. 6, 1 June 2019 (2019-06-01), pages 1514-1525 describes an arteriolar pulse transit time "PTT" based method for beat-to-beat blood pressure "BP" measurement. The proposed method can provide accurate measurements of systemic vascular resistance "SVR" and BP, which are traditionally difficult to measure in a noninvasive or continuous fashion.

There is, however, still a need for techniques for measuring flow resistances of blood vessels quickly and cost effectively.

### Summary

The following presents a simplified summary in order to provide a basic understanding of some aspects of various invention embodiments. The summary is not an extensive overview of the invention. It is neither intended to identify key or critical elements of the invention nor to delineate the scope of the invention. The following summary merely presents some concepts of the invention in a simplified form as a prelude to a more detailed description of exemplifying embodiments of the invention.

In accordance with the invention, there is provided a new apparatus for measuring flow resistances of blood vessels. An apparatus according to the invention comprises:
- a photoplethysmography "PPG" sensor configured to emit electromagnetic radiation to the blood vessels, to receive a part of the electromagnetic radiation reflected off the blood vessels, and to produce a measurement signal indicative of the received part of the electromagnetic radiation so that the electromagnetic radiation has different wavelengths and the measurement signal comprises wavelength-specific component signals indicative of received wavelengths reflected off the blood vessels, where a shorter one of the wavelengths is reflected off smaller ones of the blood vessels than a longer one of the wavelengths,
- a pressure instrument configured to produce controllable mechanical pressure applied on the blood vessels, and
- a control system configured to find, from each of the wavelength-specific component signals, a portion whose envelope, i.e. a curve outlining extremes of the wavelength-specific component signal, has exponential change, i.e. exponential growth or exponential decrease, with respect to time when the mechanical pressure changes, i.e. decreases or increases, linearly with respect to time, and to produce, for each of the wavelength-specific component signals, an estimate for a coefficient of time related to the exponential change, the coefficients of time being indicative of compliances of the blood vessels reflecting off the different wavelengths.

The control system is configured to optimize resistance values of a circuit model to minimize differences between waveforms of node voltages of the circuit model and measured waveforms of blood pressures being in the blood vessels reflecting off different ones of the wavelengths, wherein capacitor values of the circuit model are based on the above-mentioned compliances of the blood vessels reflecting off the different wavelengths and the optimized resistance values are indicative of the flow resistances of the blood vessels reflecting off the different wavelengths.

The above-mentioned pressure instrument can be for example a device for directing mechanical pressure to a fingertip or a toe, or a device comprising a cuff and a pump system for controlling gas pressure inside the cuff to direct mechanical pressure to an arm.

The photoplethysmography "PPG" sensor can be configured to emit electromagnetic radiation having wavelength in the range from 625 nm to 1000 nm, i.e. red or infrared light, in order to measure the flow resistance of arteries located in the hypodermis, and electromagnetic radiation having wavelength in the range from 565 nm to 590 nm, i.e. yellow light, in order to measure the flow resistance of blood vessels located in an upper portion of the hypodermis, and electromagnetic radiation having wavelength in the range from 500 nm to 565 nm, i.e. green light, in order to measure the flow resistance of arterioles located in the dermis, and electromagnetic radiation having wavelength in the range from 450 nm to 485 nm, i.e. blue light, in order to measure the flow resistance of capillaries located in an upper portion of the dermis.

In accordance with the invention, there is provided also a new method for measuring flow resistances of blood vessels. A method according to the invention comprises:
- emitting electromagnetic radiation to the blood vessels so that the electromagnetic radiation has different wavelengths,
- receiving a part of the electromagnetic radiation reflected off the blood vessels,
- producing a measurement signal indicative of the received part of the electromagnetic radiation so that the measurement signal comprises wavelength-specific component signals indicative of received wavelengths reflected off the blood vessels, where a shorter one of the wavelengths is reflected off smaller ones of the blood vessels than a longer one of the wavelengths,
- producing mechanical pressure applied on the blood vessels,
- finding, from each of the wavelength-specific component signals, a portion whose envelope has exponential change with respect to time when the mechanical pressure changes linearly with respect to time,
- producing, for each of the wavelength-specific component signals, an estimate for a coefficient of time related to the exponential change, the coefficient of time being indicative of compliance of the blood vessels reflecting off the wavelength under consideration, and
- optimizing resistance values of a circuit model to minimize differences between waveforms of node voltages of the circuit model and measured waveforms of blood pressures being in the blood vessels reflecting off different ones of the wavelengths, wherein capacitor values of the circuit model are based on the compliances of the blood vessels and the optimized resistance values are indicative of the flow resistances of the blood vessels.

In accordance with the invention, there is provided also a new computer program for measuring flow resistances of blood vessels. A computer program according to the invention comprises computer executable instructions for controlling a programmable processing system to:
- control a photoplethysmography sensor to emit electromagnetic radiation to the blood vessels, to receive a part of the electromagnetic radiation reflected off the blood vessels, and to produce a measurement signal indicative of the received part of the electromagnetic radiation so that the electromagnetic radiation has different wavelengths and the measurement signal comprises wavelength-specific component signals indicative of received wavelengths reflected off the blood vessels, where a shorter one of the wavelengths is reflected off smaller ones of the blood vessels than a longer one of the wavelengths,
- control a pressure instrument to produce mechanical pressure applied on the blood vessels,
- control the programmable processing system to find, from each of the wavelength-specific component signals, a portion whose envelope has exponential change with respect to time when the mechanical pressure changes linearly with respect to time, and produce, for each of the wavelength-specific component signals, an estimate for a coefficient of time related to the exponential change, the coefficient of time being indicative of compliance of the blood vessels reflecting off the wavelength under consideration, and
- optimize resistance values of a circuit model to minimize differences between waveforms of node voltages of the circuit model and measured waveforms of blood pressures being in the blood vessels reflecting off different ones of the wavelengths, wherein capacitor values of the circuit model are based on the compliances of the blood vessels and the optimized resistance values are indicative of the flow resistances of the blood vessels.

In accordance with the invention, there is provided also a new computer program product. A computer program product according to the invention comprises a nonvolatile computer readable medium, e.g. a compact disc "CD", encoded with a computer program according to the invention.

Exemplifying and non-limiting embodiments are described in accompanied dependent claims.

Various exemplifying and non-limiting embodiments both as to constructions and to methods of operation, together with additional objects and advantages thereof, will be best understood from the following description of specific exemplifying embodiments when read in conjunction with the accompanying drawings.

The verbs "to comprise" and "to include" are used in this document as open limitations that neither exclude nor require the existence of also un-recited features.

The features recited in the accompanied dependent claims are mutually freely combinable unless otherwise explicitly stated.

Furthermore, it is to be understood that the use of "a" or "an", i.e. a singular form, throughout this document does not exclude a plurality.

### Brief description of figures

Exemplifying and non-limiting embodiments and their advantages are explained in greater detail below with reference to the accompanying drawings, in which:
figure 1a illustrates an apparatus according to an exemplifying and non-limiting embodiment for measuring flow resistances of blood vessels,
figure 1b shows exemplifying graphs illustrating electromagnetic radiations having different wavelengths and reflected off blood vessels of a fingertip as a function of time,
figure 1c shows the exemplifying graphs of figure 1b converted to a logarithmic vertical scale,
figure 1d shows an exemplifying circuit model used for estimating the flow resistances of blood vessels,
figure 2 illustrates an apparatus according to an exemplifying and non-limiting embodiment for measuring flow resistances of blood vessels, and
figure 3 shows a flowchart of a method according to an exemplifying and non-limiting embodiment for measuring flow resistances of blood vessels.

### Description of exemplifying and non-limiting embodiments

The specific examples provided in the description below should not be construed as limiting the scope and/or the applicability of the appended claims. Lists and groups of examples provided in the description are not exhaustive unless otherwise explicitly stated.

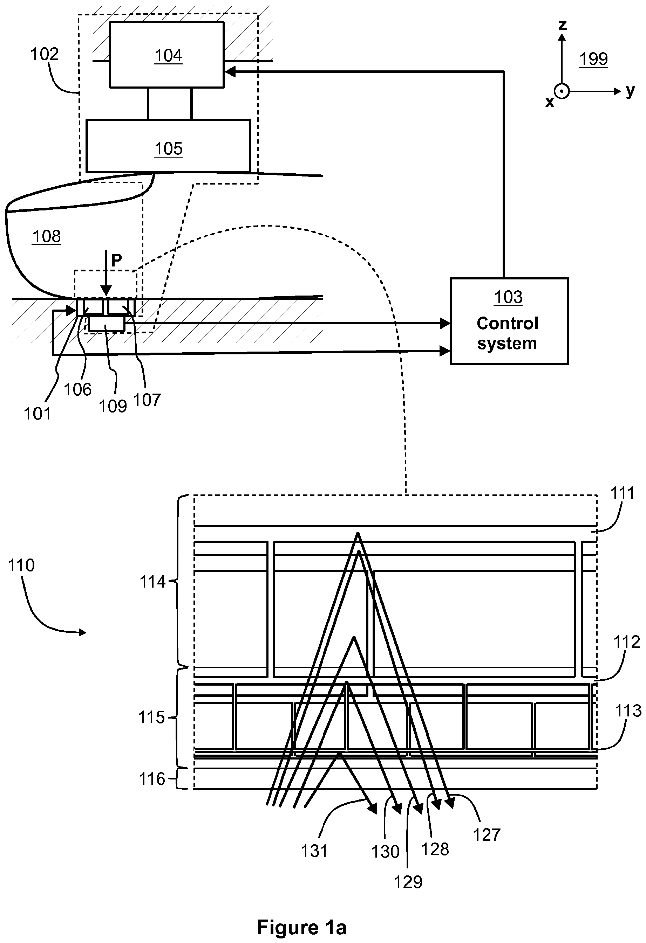

Figure 1a shows a schematic illustration of an apparatus according to an exemplifying and non-limiting embodiment for measuring flow resistances of blood vessels. The apparatus comprises a photoplethysmography "PPG" sensor 101 for emitting, to a fingertip 108 of an individual, electromagnetic radiation and for receiving a part of the electromagnetic radiation reflected off the blood vessels of the fingertip 108. The PPG sensor 101 comprises a radiation emitter 106 and a photodetector 107. The radiation emitter 106 may comprises e.g. one or more light emitting diodes "LED" and the photodetector 107 may comprise e.g. one or more photodiodes or phototransistors. Figure 1a shows also a magnified, schematic section view 110 of the fingertip. The section plane is parallel with the yz-plane of a coordinate system 199.

In the exemplifying apparatus illustrated in figure 1a, the PPG sensor 101 is configured to emit the electromagnetic radiation so that the electromagnetic radiation contains radiation components with five different wavelengths. In the section view 110, the radiation components are depicted with polyline arrows 127, 128, 129, 130, and 131. The first radiation component 127 can be for example infrared radiation having a wavelength on the range from 700 nm to 1000 nm, the second radiation component 128 can be for example red radiation having a wavelength on the range from 625 nm to 700 nm, the third radiation component 129 can be for example yellow radiation having a wavelength on the range from 565 nm to 590 nm, the fourth radiation component 130 can be for example green radiation having a wavelength on the range from 500 nm to 565 nm, and the fifth radiation component 131 can be for example blue radiation having a wavelength on the range from 450 nm to 485 nm. For another example, the first radiation component 127 can be infrared radiation having a wavelength on the range from 800 nm to 900 nm, the second radiation component 128 can be red radiation having a wavelength on the range from 650 nm to 675 nm, the third radiation component 129 can be yellow radiation having a wavelength on the range from 575 nm to 580 nm, the fourth radiation component 130 can be green radiation having a wavelength on the range from 530 nm to 545 nm, and the fifth radiation component 131 can be blue radiation having a wavelength on the range from 460 nm to 475 nm.

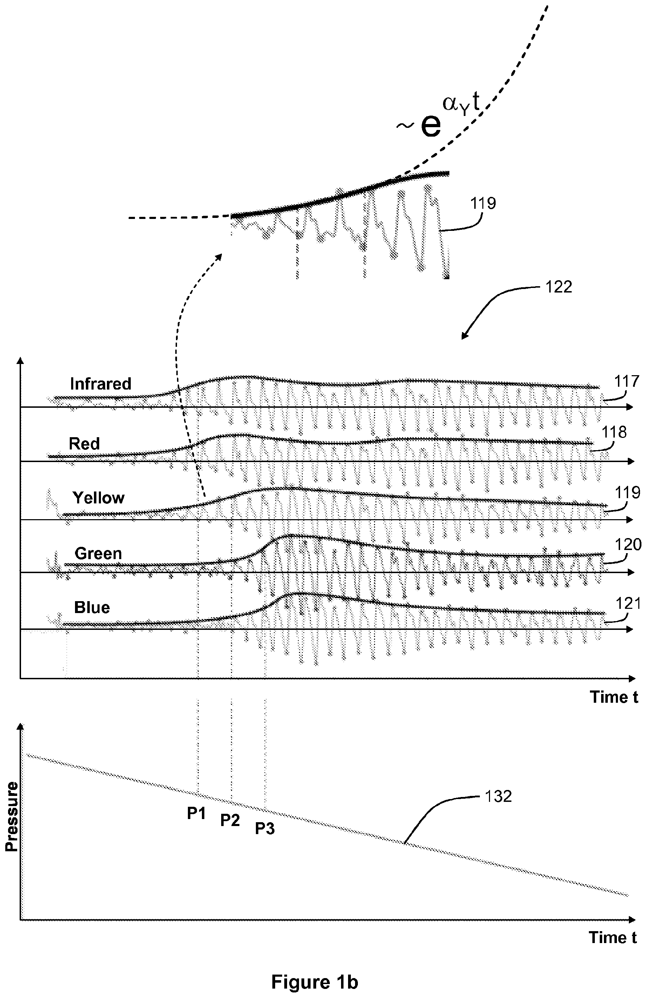

As illustrated in the section view 130, the red and infrared radiation components 127 and 128 reach arteries 111 located in the hypodermis 114, the yellow radiation component 129 reach blood vessels located in a portion of the hypodermis 114 adjacent to the dermis 115, the green radiation component 130 reach arterioles 112 located in the dermis 115, and the blue radiation component 131 reach capillaries 112 located in a portion of the dermis 115 adjacent to the epidermis 116. Therefore, shorter wavelengths are reflected off smaller blood vessels, i.e. blood vessels nearer to a skin surface, than longer wavelengths. The photodetector 107 of the PPG sensor 101 is configured produce a measurement signal that comprises wavelength-specific component signals indicative of received wavelengths reflected off the blood vessels. Figure 1b shows graphs illustrating exemplifying wavelength-specific component signals 117, 118, 119, 120, and 121 of an exemplifying measurement signal 122. The wavelength-specific component signal 117 corresponds to infrared radiation, the wavelength-specific component signal 118 corresponds to red radiation, the wavelength-specific component signal 119 corresponds to yellow radiation, the wavelength-specific component signal 120 corresponds to green radiation, and the wavelength-specific component signal 121 corresponds to blue radiation. The photodetector 107 may comprise for example many photodiodes or phototransistors that are sensitive to different wavelengths, or the photodetector 107 may comprise filters to implement wavelength separation.

The apparatus comprises a pressure instrument 102 configured to produce controllable mechanical pressure P applied on the blood vessels. The apparatus comprises a control system 103 configured to control the pressure instrument 102 to change, i.e. to decrease or increase, the mechanical pressure linearly with respect to time t when the electromagnetic radiation is emitted to the blood vessels and a reflected part of the electromagnetic radiation is received from the blood vessels. Figure 1b shows a line 132 illustrating the time dependence of the mechanical pressure in the exemplifying situation in which the mechanical pressure has been linearly decreased and the above-mentioned wavelength-specific component signals 117-121 have been measured.

In the exemplifying apparatus illustrated in figure 1a, the pressure instrument 102 comprises a pressure sensor 109 for measuring the mechanical pressure P directed by the fingertip 108 to the pressure sensor 108 and pressing means for controllably pressing the fingertip 108 against the PPG sensor 101 and the pressure sensor 109. In this exemplifying apparatus, the pressing means comprise a pressing element 105 and a force generator 104 for directing force to the pressing element 105. The force generator 104 may comprise for example an electric stepper motor and a threaded rod or some other suitable elements for generating force.

The control system 103 is configured to find, from each of the wavelength-specific component signals 117-121, a portion whose envelope has exponential change, i.e. exponential growth or exponential decrease, with respect to time when the mechanical pressure decreases linearly with respect to time. The control system 103 is configured to produce, for each of the wavelength-specific component signals 117-121, an estimate for a coefficient of time related to the above-mentioned exponential change. The coefficient of time is indicative of the compliance of the blood vessels reflecting off the wavelength corresponding to the wavelength-specific component signal under consideration and thereby also the stiffness of these blood vessels.

As mentioned above, in the exemplifying case illustrated in figure 1b, the mechanical pressure is linearly decreased and thus each of the wavelength-specific component signals 117-121 has a portion whose envelope has exponential growth. Figure 1b shows a magnification of a part of the wavelength-specific component signal 119. In figure 1b, the envelopes of the wavelength-specific component signals 117-121 are depicted with thick lines. The exponential growth ~e^{αYt} on a part of the envelope of the wavelength-specific component signal 119 is depicted with a dashed line. In this case, the coefficient of time related to the exponential growth is α_{Y}. Thus, the coefficient of time α_{Y} is indicative of the compliance of the blood vessels from which yellow light is reflected off and thereby also the stiffness of the blood vessels from which the yellow light is reflected off.

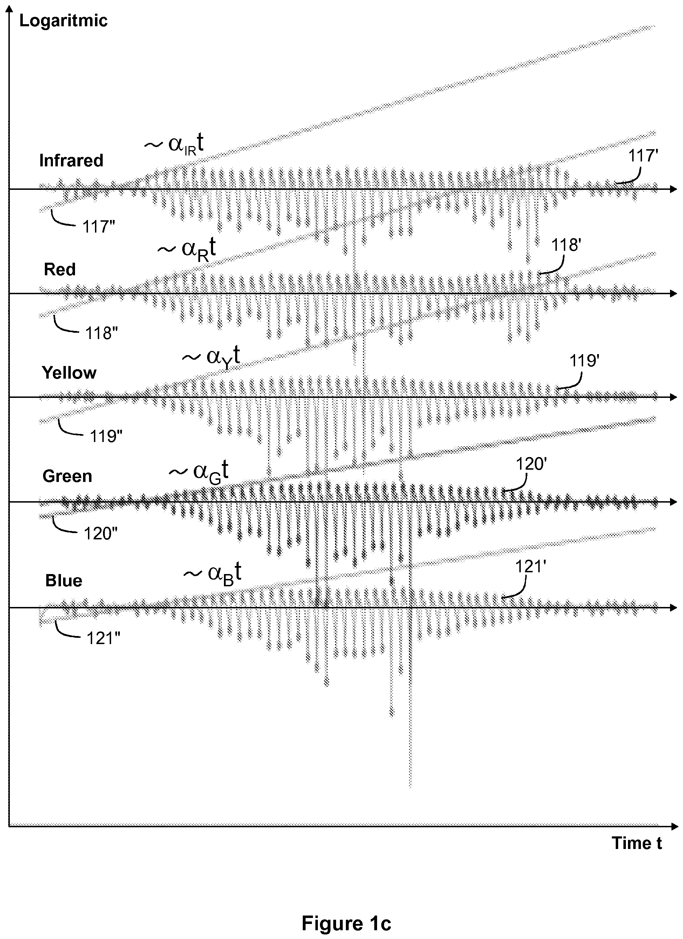

There are many ways to find the portion whose envelope has the exponential change when the mechanical pressure changes linearly and to produce the estimate for the coefficient of time related to the exponential change. For example, curve fitting based on e.g. the least-mean-square "LMS" method can be used. Thus, apparatuses according to embodiments of the invention are not limited to any specific ways to find the portion whose envelope has the exponential change and to produce the estimate for the coefficient of time related to the exponential change. For example, in an apparatus according to an exemplifying and non-limiting embodiment, the control system 103 is configured to convert the wavelength-specific components 117-121 of the measurement signal to a logarithmic scale. Figure 1c shows graphs illustrating the converted wavelength-specific component signals 117', 118', 119', 120', and 121' which corresponds to the wavelength-specific component signals 117, 118, 119, 120, and 121 shown in figure 1b, respectively. Due to the logarithmic conversion, the exponential change is converted into linear change that is depicted with lines 117", 118", 119", 120", and 121" shown in figure 1c. The control system 103 is configured to estimate the slope of the linear change of the envelope of each converted wavelength-specific component signal. The slope is the coefficient of time related to the exponential change. In the exemplifying case shown in figures 1b and 1c, the slope i.e. the coefficient of time related to the infrared light is α_{IR}, the coefficient of time related to the red light is α_{R}, the coefficient of time related to the yellow light is α_{Y}, the coefficient of time related to the green light is α_{G}, and the coefficient of time related to the blue light is α_{B}. Thus, for example, α_{R} is the indicative of compliance of arteries and thereby also the stiffness of the arteries, α_{G} is indicative of compliance of arterioles and thereby also the stiffness of the arterioles, and as is indicative of compliance of capillaries and thereby also the stiffness of the capillaries.

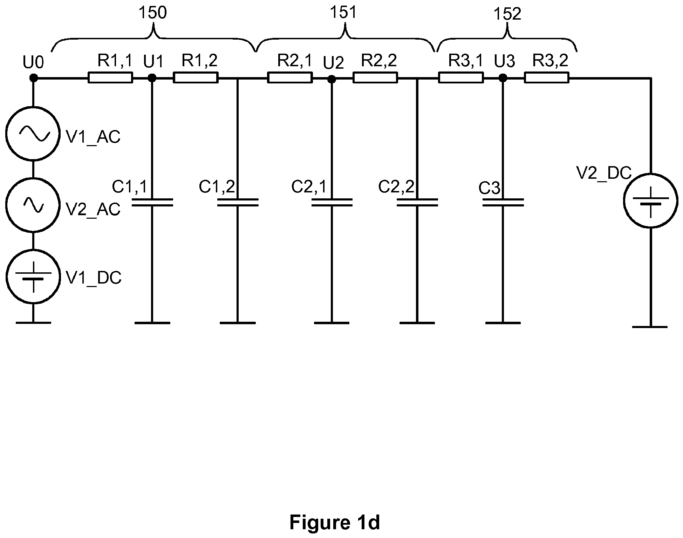

The control system 103 is configured to optimize resistance values of a circuit model to minimize differences between waveforms of node voltages of the circuit model and measured waveforms of blood pressures prevailing in the blood vessels reflecting off different ones of the wavelengths. Capacitor values of the circuit model are based on the above-mentioned compliances of the blood vessels, and the optimized resistance values are indicative of the flow resistances of the blood vessels. Figure 1d shows an exemplifying circuit model used for estimating the flow resistances of blood vessels. In this exemplifying case, the circuit model comprises three circuit segments 150, 151, and 152. The circuit segment 150 may model for example arteries located in the hypodermis, the circuit segment 151 may model for example arterioles located in the dermis, and the circuit segment 152 may model for example capillaries located in an upper portion of the dermis. Node voltage U1 can model the blood pressure prevailing in the blood vessels reflecting off infrared and red light, node voltage U2 can model the blood pressure prevailing in the blood vessels reflecting off green light, and node voltage U3 can model the blood pressure prevailing in the blood vessels reflecting off blue light. Node voltage U0 can have a waveform whose maximum is measured systolic blood pressure and whose minimum is measured diastolic blood pressure. The shape of the waveform of the node voltage U0 can be modelled e.g. as U0 = V1_AC + V2_AC + V1_DC, where V1_AC = V1sin(2π × HR/60 × t) and V2_AC = V2sin(2π × 2HR/60 × t), where t is time in seconds, HR is a heart rate as heart beats per minute, and V1, V2, and V1_DC are selected so that the maximum of U0 is the measured systolic blood pressure and the minimum of U0 is the measured diastolic blood pressure. Voltage levels in the circuit model are advantageously selected so that volts correspond directly to millimeters of mercury "mmHg". In an exemplifying case, HR = 60/min, V1 = 10 volts, V2 = 10 volts, and V1_DC = from 80 to 120 volts, e.g. 100 volts. In this exemplifying case, the peak-to-peak of U0 is from 25 to 40 volts e.g. about 35 V. Voltage V2_DC models the blood pressure after the capillaries and it can be on the range from 0 to 10 volts, or on the range from 3 to 8 volts. As mentioned above, the capacitor values of the circuit model are based on the above-mentioned compliances of the blood vessels. In this exemplifying case, the capacitor values of the circuit model can be for example: C1,1 = C1,2 = α_{IR}/2 or α_{R}/2, C2,1 = C2,2 = α_{G}/2, and C3 = α_{B}.

It is to be noted that the above-described way to model the input voltage U0 of the circuit model is an example only. It is also possible to measure or estimate a waveform of blood pressure at the beginning of the greatest blood vessels, e.g. arteries, modelled by the circuit model and thereafter use the measured or estimated blood pressure waveform as the input voltage U0 of the circuit model. Furthermore, characteristic values e.g. amplitudes and phases of different sinusoidal AC components and a DC component of the input voltage U0 can be optimized parameters along with the resistance values of the circuit model. Furthermore, instantaneous values forming the waveform the input voltage U0 can be optimized parameters along with the resistance values of the circuit model. Thus, the invention is not limited to any specific ways to obtain the input voltage U0 of the circuit model. The measurements are advantageously calibrated so that e.g. the measured pressure values correspond directly to voltage values of the circuit model.

In an apparatus according to an exemplifying and non-limiting embodiment, the control system 103 is configured to variate the resistance values R1,1, R1,2, R2,1, R2,2, R3,1, and R3,2 as long as a sum of time integrals of squares of differences between the waveforms of the node voltages U1, U2, and U3 and the measured waveforms of the blood pressures is decreasing. The optimized resistance values are indicative of the flow resistances of the blood vessels reflecting off different wavelengths. In the above-described exemplifying case, R1,1 + R1,2 is the flow resistance of the arteries, R2,1 + R2,2 is the flow resistance of the arterioles, and R3,1 + R3,2 is the flow resistance of the capillaries. The resistance values can be varied e.g. stochastically in which case the optimization process is a simulated evolution process. It is to be noted that any suitable multivariable optimization technique can be used, and the invention is not limited to any specific multivariable optimization techniques. Furthermore, it is to be noted that the circuit model shown in figure 1d is a non-limiting example only, and different circuit models can be used in conjunction with different embodiments of the invention. For example, blood vessels reflecting off a given wavelength, such as e.g. arteries, can be modelled with only one resistor-capacitor "RC" loop or with three or more subsequently connected RC-loops.

The waveforms of the blood pressures prevailing in the blood vessels reflecting off different wavelengths can be measured for example in the following way: The maximum value of blood pressure prevailing in blood vessels reflecting off a given wavelength is a value of a down ramping pressure, e.g. the pressure 132 in figure 1b, at which the corresponding wavelength-specific component signal 117, 118, 119, 120, or 121 has 50 % of its maximum peak-to-peak value. For example, in the exemplifying case shown in figure 1b, the maximum value of the blood pressure prevailing in blood vessels reflecting off infrared and red light is P1, the maximum value of the blood pressure prevailing in blood vessels reflecting off yellow light is P2, and the maximum value of the blood pressure prevailing in blood vessels reflecting off green light is P3. The pressure values P1, P2, and P3 can be e.g. 127 mmHg, 100 mmHg, and 81 mmHg, respectively. The mean value of blood pressure prevailing in blood vessels reflecting off a given wavelength is a value of the down ramping pressure 132 at which the corresponding wavelength-specific component signal 117, 118, 119, 120, or 121 has its maximum peak-to-peak value. The shape of the waveform of blood pressure prevailing in blood vessels reflecting off a given wavelength is the shape of the corresponding wavelength-specific component signal 117, 118, 119, 120, or 121 when the mechanical pressure is kept constant, advantageously zero. The waveform of each wavelength-specific component signal can then be scaled and shifted so that its maximum value is the above-mentioned maximum value and its mean value is the above-mentioned mean value. More information about ways to measure the waveforms of blood pressures prevailing in blood vessels reflecting off different wavelengths can be found e.g. from the publication Panula, T., et al.: "An instrument for measuring blood pressure and assessing cardiovascular health from the fingertip", Biosensors and Bioelectronics, Volume 167, 1 November 2020, 112483. It is to be noted that the invention is not limited to any specific way to measure the waveforms of the blood pressures prevailing in the blood vessels reflecting off different wavelengths.

In the exemplifying apparatus illustrated in figure 1a, the PPG sensor 101 is configured to emit and receive different wavelengths simultaneously. It is however also possible that the control system of an apparatus according to an exemplifying and non-limiting embodiment is configured to control the PPG sensor to variate the wavelength of the electromagnetic radiation, and to produce successively the coefficients of time, such as α_{IR}, α_{R}, α_{Y}, α_{G}, and α_{B}, for different wavelengths as well as the waveforms of the blood pressures prevailing in the blood vessels reflecting off different wavelengths.

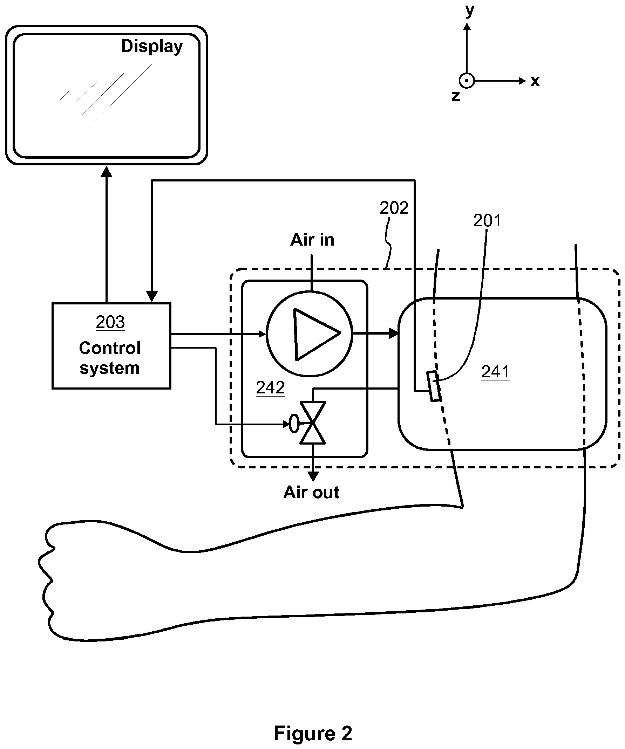

Figure 2 shows a schematic illustration of an apparatus according to an exemplifying and non-limiting embodiment for measuring flow resistances of blood vessels. The apparatus comprises a photoplethysmography "PPG" sensor 201 for emitting electromagnetic radiation having different wavelengths to blood vessels of an arm and for receiving a part of the electromagnetic radiation reflected off the blood vessels. The PPG sensor 201 is configured to produce a measurement signal indicative of the received part of the electromagnetic radiation so that the measurement signal comprises wavelength-specific component signals indicative of received wavelengths reflected off the blood vessels, where a shorter one of the wavelengths is reflected off smaller ones of the blood vessels than a longer one of the wavelengths.

The apparatus comprises a pressure instrument 202 configured to produce controllable mechanical pressure applied on the blood vessels. The apparatus comprises a control system 203 configured to control the pressure instrument 202 to decrease the mechanical pressure linearly with respect to time when the electromagnetic radiation is emitted to the blood vessels and the reflected electromagnetic radiation is received from the blood vessels. The control system 203 is configured to find, from each wavelength-specific component signal, a portion whose envelope has exponential change with respect to time when the mechanical pressure changes linearly with respect to time and to produce, for each wavelength-specific component signal, an estimate for a coefficient of time related to the exponential change. The coefficient of time is indicative of the compliance of the blood vessels reflecting off the wavelength under consideration, and thereby the coefficient of time is indicative of the stiffness of the blood vessels. The control system 203 is configured to optimize resistance values of a circuit model to minimize differences between waveforms of node voltages of the circuit model and measured waveforms of blood pressures being in the blood vessels reflecting off different ones of the wavelengths. Capacitor values of the circuit model are based on the above-mentioned compliances of the blood vessels, and the optimized resistance values are indicative of the flow resistances of the blood vessels.

In the exemplifying apparatus illustrated in figure 2, the pressure instrument 202 comprises a cuff and 241 a pump system 242 configured to control gas pressure inside the cuff and thereby to control the mechanical pressure directed to the blood vessels when the PPG sensor 201 emits and receives the electromagnetic radiation to and from the arm. The PPG sensor is located on an inner surface of the cuff.

Each of the control systems 103 and 203 shown in figures 1a and 2 can be implemented for example with one or more processor circuits, each of which can be a programmable processor circuit provided with appropriate software, a dedicated hardware processor such as for example an application specific integrated circuit "ASIC", or a configurable hardware processor such as for example a field programmable gate array "FPGA". Each of the control systems 103 and 203 may further comprise memory implemented for example with one or more memory circuits each of which can be e.g. a random-access memory "RAM" device.

An apparatus according to an exemplifying and non-limiting embodiment for measuring flow resistances of blood vessels comprises a control system configured to:
a) estimate cardiac output "CO" according to the formula CO = stroke volume "SV" × Heart rate "HR" × α_{C} × (√(R_{cr}(0)) + √(-R_{cr}(τₘᵢₙ))), where R_{cr} is the autocorrelation of the arterial blood pressure waveform, τₘᵢₙ is the smallest time shift in the autocorrelation that gives a peak in the autocorrelation which is comparable to the value R_{cr}(0) at zero shift in the autocorrelation, and α_{C} is a calibration constant that can be determined e.g. experimentally,
b) estimate the mean arterial pressure "MAP" with a suitable known method,
c) compute systemic vascular resistance "SVR" = MAP/CO,
d) compute the blood flow through the vessels F = blood pressure at arteries / SVR, and
e) compute the flow resistance R at a given point in the vasculature, e.g. arteries, arterioles, or capillaries = the blood pressure at the given point / F.

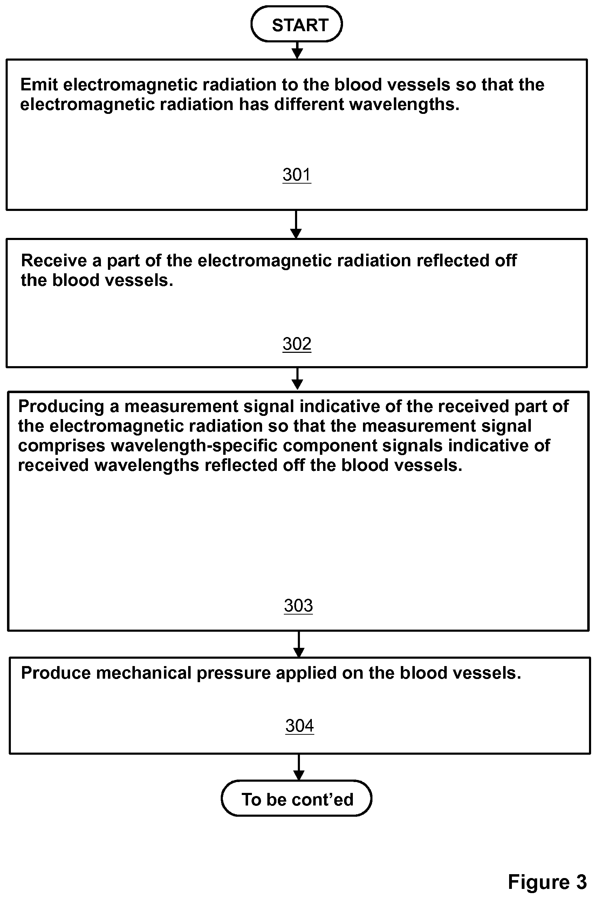

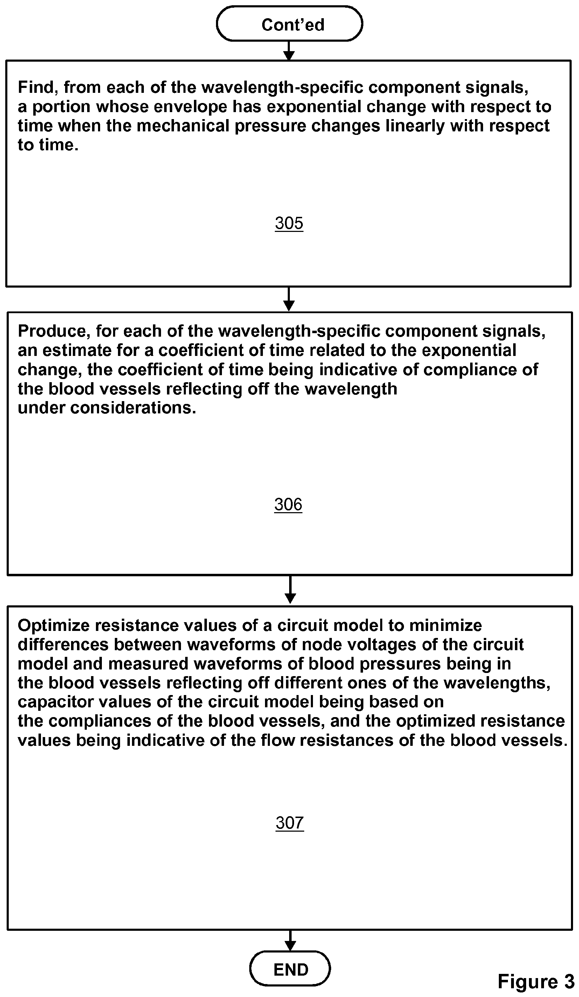

Figure 3 shows a flowchart of a method according to an exemplifying and non-limiting embodiment for measuring flow resistances of blood vessels. The method comprises the following actions:
- action 301: emitting electromagnetic radiation to the blood vessels so that the electromagnetic radiation has different wavelengths,
- action 302: receiving a part of the electromagnetic radiation reflected off the blood vessels,
- action 303: producing a measurement signal indicative of the received part of the electromagnetic radiation so that the measurement signal comprises wavelength-specific component signals indicative of received wavelengths reflected off the blood vessels, where a shorter one of the wavelengths is reflected off smaller ones of the blood vessels than a longer one of the wavelengths,
- action 304: producing mechanical pressure applied on the blood vessels,
- action 305: finding, from each of the wavelength-specific component signals, a portion whose envelope has exponential change with respect to time when the mechanical pressure changes linearly with respect to time,
- action 306: producing, for each of the wavelength-specific component signals, an estimate for a coefficient of time related to the exponential change, the coefficient of time being indicative of compliance of the blood vessels reflecting off the wavelength under consideration, and
- action 307: optimizing resistance values of a circuit model to minimize differences between waveforms of node voltages of the circuit model and measured waveforms of blood pressures prevailing in the blood vessels reflecting off different ones of the wavelengths, wherein capacitor values of the circuit model are based on the compliances of the blood vessels, and the optimized resistance values are indicative of the flow resistances of the blood vessels.

In a method according to an exemplifying and non-limiting embodiment, the electromagnetic radiation has wavelengths selected from the following ranges: from 625 nm to 1000 nm, from 565 nm to 590 nm, from 500 nm to 565 nm, and from 450 nm to 485 nm.

A method according to an exemplifying and non-limiting embodiment comprises converting the measurement signal to a logarithmic scale, finding from the converted measurement signal a portion whose envelope has linear change with respect to time, and producing an estimate for a slope of the envelope of the converted measurement signal related to the linear change. The slope of the linear change is the coefficient of time related to the exponential change.

In a method according to an exemplifying and non-limiting embodiment, the mechanical pressure is directed to a fingertip or a toe of an individual.

In a method according to an exemplifying and non-limiting embodiment, the mechanical pressure is directed to an arm of an individual with a cuff and a pump system configured to control gas pressure inside the cuff. In this exemplifying case, the photoplethysmography sensor is located on an inner surface of the cuff.

In a method according to an exemplifying and non-limiting embodiment, the resistance values of the circuit model are optimized so that the resistance values are variated to as long as a sum of time integrals of squares of differences between the waveforms of the node voltages and the measured waveforms of the blood pressures is decreasing.

A method according to an exemplifying and non-limiting embodiment for measuring flow resistances of blood vessels comprises the following:
a) estimating cardiac output "CO" according to the formula CO = stroke volume "SV" × Heart rate "HR" × α_{C} × (√(R_{cr}(0)) + √(-R_{cr}(τₘᵢₙ))), where R_{cr} is the autocorrelation of the arterial blood pressure waveform, τₘᵢₙ is the smallest time shift in the autocorrelation that gives a peak in the autocorrelation which is comparable to the value R_{cr}(0) at zero shift in the autocorrelation, and α_{C} is a calibration constant that can be determined e.g. experimentally,
b) estimating the mean arterial pressure "MAP" with a suitable known method,
c) computing systemic vascular resistance "SVR" = MAP/CO,
d) computing the blood flow through the vessels F = blood pressure at arteries / SVR, and
e) computing the flow resistance R at a given point in the vasculature, e.g. arteries, arterioles, or capillaries = the blood pressure at the given point / F.

A computer program according to an exemplifying and non-limiting embodiment comprises computer executable instructions for controlling a programmable processing system to carry out actions related to a method according to any of the above-described exemplifying and non-limiting embodiments.

A computer program according to an exemplifying and non-limiting embodiment comprises software modules for measuring flow resistances of blood vessels. The software modules comprise computer executable instructions for controlling a programmable processing system to:
- control a photoplethysmography sensor to emit electromagnetic radiation to the blood vessels, to receive a part of the electromagnetic radiation reflected off the blood vessels, and to produce a measurement signal indicative of the received part of the electromagnetic radiation so that the electromagnetic radiation has different wavelengths and the measurement signal comprises wavelength-specific component signals indicative of received wavelengths reflected off the blood vessels, where a shorter one of the wavelengths is reflected off smaller ones of the blood vessels than a longer one of the wavelengths,
- control a pressure instrument to produce mechanical pressure applied on the blood vessels,
- control the programmable processing system to find, from each of the wavelength-specific component signals, a portion whose envelope has exponential change with respect to time when the mechanical pressure changes linearly with respect to time, and produce, for each of the wavelength-specific component signals, an estimate for a coefficient of time related to the exponential change, the coefficient of time being indicative of compliance of the blood vessels reflecting off the wavelength under consideration, and
- optimize resistance values of a circuit model to minimize differences between waveforms of node voltages of the circuit model and measured waveforms of blood pressures being in the blood vessels reflecting off different ones of the wavelengths, wherein capacitor values of the circuit model are based on the compliances of the blood vessels and the optimized resistance values are indicative of the flow resistances of the blood vessels.

The software modules can be for example subroutines or functions implemented with programming tools suitable for the programmable processing system.

A computer program product according to an exemplifying and non-limiting embodiment comprises a computer readable medium, e.g. a compact disc "CD", encoded with a computer program according to an exemplifying embodiment.

A signal according to an exemplifying and non-limiting embodiment is encoded to carry information defining a computer program according to an exemplifying embodiment.

A computer program according to an exemplifying and non-limiting embodiment may constitute e.g. a part of a software of a mobile device, e.g. a smart phone or a wearable device.

A computer program according to an exemplifying and non-limiting embodiment comprises computer executable instructions for controlling a programmable processing system to:
a) estimate cardiac output "CO" according to the formula CO = stroke volume "SV" × Heart rate "HR" × α_{C} × (√(R_{cr}(0)) + √(-R_{cr}(τₘᵢₙ))), where R_{cr} is the autocorrelation of the arterial blood pressure waveform, τₘᵢₙ is the smallest time shift in the autocorrelation that gives a peak in the autocorrelation which is comparable to the value R_{cr}(0) at zero shift in the autocorrelation, and α_{C} is a calibration constant that can be determined e.g. experimentally,
b) compute systemic vascular resistance "SVR" = MAP/CO, where MAP is the mean arterial pressure determined with a suitable known method,
c) compute the blood flow through the vessels F = blood pressure at arteries / SVR, and
d) compute the flow resistance R at a given point in the vasculature, e.g. arteries, arterioles, or capillaries = the blood pressure at the given point / F.

The specific examples provided in the description given above should not be construed as limiting the scope and/or the applicability of the appended claims. Lists and groups of examples provided in the description given above are not exhaustive unless otherwise explicitly stated.

## Claims

1. An apparatus for measuring flow resistances of blood vessels, the apparatus comprising:
- a photoplethysmography sensor (101, 201) configured to emit electromagnetic radiation to the blood vessels, to receive a part of the electromagnetic radiation reflected off the blood vessels, and to produce a measurement signal (122) indicative of the received part of the electromagnetic radiation so that the electromagnetic radiation has different wavelengths and the measurement signal comprises wavelength-specific component signals (117-121) indicative of received wavelengths reflected off the blood vessels, where a shorter one of the wavelengths is reflected off smaller ones of the blood vessels than a longer one of the wavelengths,
- a pressure instrument (102, 202) configured to produce controllable mechanical pressure applied on the blood vessels, and
- a control system (103, 203) configured to find, from each of the wavelength-specific component signals, a portion whose envelope has exponential change (~e^{αYt}) with respect to time (t) when the mechanical pressure changes linearly with respect to time, and produce, for each of the wavelength-specific component signals, an estimate for a coefficient (α_{IR}, α_{R}, α_{Y}, α_{G}, α_{B}) of time related to the exponential change, the coefficient of time being indicative of compliance of the blood vessels reflecting off the wavelength under consideration,
wherein the control system is configured to optimize resistance values of a circuit model to minimize differences between waveforms of node voltages of the circuit model and measured waveforms of blood pressures being in the blood vessels reflecting off different ones of the wavelengths, wherein capacitor values of the circuit model are based on the compliances of the blood vessels and the optimized resistance values are indicative of the flow resistances of the blood vessels.

2. An apparatus according to claim 1, wherein the photoplethysmography sensor (101) is configured to emit, to the blood vessels, the electromagnetic radiation so that the electromagnetic radiation has the wavelengths selected from the following ranges: from 625 nm to 1000 nm, from 565 nm to 590 nm, from 500 nm to 565 nm, and from 450 nm to 485 nm.

3. An apparatus according to any claim 1 or 2, wherein the control system (103) is configured to convert the wavelength-specific component signals to a logarithmic scale, to find from each converted wavelength-specific component signal a portion whose envelope has linear change with respect to time, and to produce an estimate for a slope (α_{IR}, α_{R}, α_{Y}, α_{G}, α_{B}) of the envelope of the converted wavelength-specific component signal related to the linear change, the slope being the coefficient of time related to the exponential change of the wavelength-specific component signal under consideration.

4. An apparatus according to any one of claims 1-3, wherein the pressure instrument (102) comprises a force generator (104) and a pressing element (105) configured to direct the mechanical pressure to a fingertip or a toe in accordance with a control signal generated by the control system (103).

5. An apparatus according to any one of claims 1-3, wherein the pressure instrument (202) comprises a cuff (241) and a pump system (242) configured to control gas pressure inside the cuff to direct the mechanical pressure to an arm.

6. An apparatus according to any one of claims 1-5, wherein the control system (103) is configured to variate the resistance values to as long as a sum of time integrals of squares of differences between the waveforms of the node voltages and the measured waveforms of blood pressures is decreasing.

7. A method for measuring flow resistances of blood vessels, the method comprising:
- emitting (301) electromagnetic radiation to the blood vessels so that the electromagnetic radiation has different wavelengths,
- receiving (302) a part of the electromagnetic radiation reflected off the blood vessels,
- producing (303) a measurement signal indicative of the received part of the electromagnetic radiation so that the measurement signal comprises wavelength-specific component signals indicative of received wavelengths reflected off the blood vessels, where a shorter one of the wavelengths is reflected off smaller ones of the blood vessels than a longer one of the wavelengths,
- producing (304) mechanical pressure applied on the blood vessels,
- finding (305), from each of the wavelength-specific component signals, a portion whose envelope has exponential change (~e^{αYt}) with respect to time when the mechanical pressure changes linearly with respect to time, and
- producing (306), for each of the wavelength-specific component signals, an estimate for a coefficient (α_{IR}, α_{R}, α_{Y}, α_{G}, α_{B}) of time related to the exponential change, the coefficient of time being indicative of compliance of the blood vessels reflecting off the wavelength under consideration,
wherein the method comprises optimizing (307) resistance values of a circuit model to minimize differences between waveforms of node voltages of the circuit model and measured waveforms of blood pressures being in the blood vessels reflecting off different ones of the wavelengths, wherein capacitor values of the circuit model are based on the compliances of the blood vessels and the optimized resistance values are indicative of the flow resistances of the blood vessels.

8. A method according to claim 7, wherein the electromagnetic radiation has wavelengths selected from the following ranges: from 625 nm to 1000 nm, from 565 nm to 590 nm, from 500 nm to 565 nm, and from 450 nm to 485 nm.

9. A method according to any claim 7 or 8, wherein the method comprises converting the wavelength-specific component signals to a logarithmic scale, finding from each converted wavelength-specific component signal a portion whose envelope has linear change with respect to time, and producing an estimate for a slope (α_{IR}, α_{R}, α_{Y}, α_{G}, α_{B}) of the envelope of the converted wavelength-specific component signal related to the linear change, the slope being the coefficient of time related to the exponential change of the wavelength-specific component signal under consideration.

10. A method according to any one of claims 7-9, wherein the mechanical pressure is directed to a fingertip or a toe.

11. A method according to any one of claims 7-10, wherein the method comprises variating the resistance values to as long as a sum of time integrals of squares of differences between the waveforms of the node voltages and the measured waveforms of the blood pressures is decreasing.

12. A computer program for measuring flow resistances of blood vessels, the computer program comprising computer executable instructions for controlling a programmable processing system to:
- control a photoplethysmography sensor to emit electromagnetic radiation to the blood vessels, to receive a part of the electromagnetic radiation reflected off the blood vessels, and to produce a measurement signal indicative of the received part of the electromagnetic radiation so that the electromagnetic radiation has different wavelengths and the measurement signal comprises wavelength-specific component signals indicative of received wavelengths reflected off the blood vessels, where a shorter one of the wavelengths is reflected off smaller ones of the blood vessels than a longer one of the wavelengths,
- control a pressure instrument to produce mechanical pressure applied on the blood vessels, and
- control the programmable processing system to find, from each of the wavelength-specific component signals, a portion whose envelope has exponential change (~e^{αYt}) with respect to time (t) when the mechanical pressure changes linearly with respect to time, and produce, for each of the wavelength-specific component signals, an estimate for a coefficient (α_{IR}, α_{R}, α_{Y}, α_{G}, α_{B}) of time related to the exponential change, the coefficient of time being indicative of compliance of the blood vessels reflecting off the wavelength under consideration,
wherein the computer program further comprises computer executable instructions for controlling the programmable processing system to optimize resistance values of a circuit model to minimize differences between waveforms of node voltages of the circuit model and measured waveforms of blood pressures being in the blood vessels reflecting off different ones of the wavelengths, wherein capacitor values of the circuit model are based on the compliances of the blood vessels and the optimized resistance values are indicative of the flow resistances of the blood vessels.

13. A computer program product comprising a non-transitory computer readable medium encoded with a computer program according to claim 12.

## Patentansprüche

1. Vorrichtung zum Messen von Strömungswiderständen von Blutgefäßen, wobei die Vorrichtung umfasst:
- einen Photoplethysmographiesensor (101, 201), der konfiguriert ist, um elektromagnetische Strahlung an die Blutgefäße auszusenden, einen Teil der von den Blutgefäßen reflektierten elektromagnetischen Strahlung zu empfangen und ein Messsignal (122) als Indikator für den empfangenen Teil der elektromagnetischen Strahlung zu erzeugen, derart, dass die elektromagnetische Strahlung unterschiedliche Wellenlängen aufweist und das Messsignal wellenlängenspezifische Komponentensignale (117-121) als Indikator für die empfangenen, von den Blutgefäßen reflektierten Wellenlängen umfasst, wobei eine kürzere der Wellenlängen von kleineren Blutgefäßen reflektiert wird als eine längere der Wellenlängen,
- ein Druckinstrument (102, 202), das konfiguriert ist, um einen steuerbaren mechanischen Druck zu erzeugen, der auf die Blutgefäße ausgeübt wird, und
- ein Steuerungssystem (103, 203), das konfiguriert ist, um in jedem der wellenlängenspezifischen Komponentensignale einen Abschnitt zu finden, dessen Hüllkurve sich im Verhältnis zur Zeit (t) exponentiell ändert (~e^{αYt}), wenn sich der mechanische Druck im Verhältnis zur Zeit linear ändert, und für jedes der wellenlängenspezifischen Komponentensignale eine Schätzung für einen Koeffizienten (α_{IR}, α_{R}, αγ, α_{G}, α_{B}) der Zeit zu erzeugen, der mit der exponentiellen Änderung in Zusammenhang steht, wobei der Zeitkoeffizient ein Indikator für die Nachgiebigkeit der Blutgefäße ist, die die betrachtete Wellenlänge reflektieren, wobei das Steuerungssystem konfiguriert ist, um Widerstandswerte eines Kreislaufmodells zu optimieren, um Differenzen zwischen Kurven von Knotenspannungen des Kreislaufmodells und gemessenen Kurven von Blutdrücken in den Blutgefäßen, die verschiedene der Wellenlängen reflektieren, zu minimieren, wobei Kondensatorwerte des Kreislaufmodells auf der Nachgiebigkeit der Blutgefäße basieren und die optimierten Widerstandswerte ein Indikator für die Strömungswiderstände der Blutgefäße sind.

2. Vorrichtung gemäß Anspruch 1, wobei der Photoplethysmographiesensor (101) konfiguriert ist, um die elektromagnetische Strahlung derart an die Blutgefäße abzugeben, dass die elektromagnetische Strahlung Wellenlängen aufweist, die aus den folgenden Bereichen ausgewählt sind: von 625 nm bis 1000 nm, von 565 nm bis 590 nm, von 500 nm bis 565 nm und von 450 nm bis 485 nm.

3. Vorrichtung gemäß Anspruch 1 oder 2, wobei das Steuerungssystem (103) konfiguriert ist, um die wellenlängenspezifischen Komponentensignale in eine logarithmische Skala umzurechnen, in jedem umgerechneten wellenlängenspezifischen Komponentensignal einen Abschnitt zu finden, dessen Hüllkurve sich im Verhältnis zur Zeit linear ändert, und eine Schätzung für eine Steigung (α_{IR}, α_{R}, αγ, α_{G}, α_{B}) der Hüllkurve des umgerechneten wellenlängenspezifischen Komponentensignals im Verhältnis zu der linearen Änderung zu erzeugen, wobei die Steigung der Zeitkoeffizient im Zusammenhang mit der exponentiellen Änderung des betrachteten wellenlängenspezifischen Komponentensignals ist.

4. Vorrichtung nach einem der Ansprüche 1 bis 3, wobei das Druckinstrument (102) einen Kraftgenerator (104) und ein Druckelement (105) umfasst, das konfiguriert ist, um den mechanischen Druck entsprechend einem vom Steuerungssystem (103) erzeugten Steuerungssignal zu einer Fingerspitze oder einem Zeh zu leiten.

5. Vorrichtung nach einem der Ansprüche 1 bis 3, wobei das Druckinstrument (202) eine Manschette (241) und ein Pumpensystem (242) umfasst, das konfiguriert ist, um den Gasdruck im Inneren der Manschette zu regeln, um den mechanischen Druck zu einem Arm zu leiten.

6. Vorrichtung nach einem der Ansprüche 1 bis 5, wobei das Steuerungssystem (103) konfiguriert ist, um die Widerstandswerte zu variieren, so lange, wie eine Summe der Zeitintegrale der Quadrate der Differenzen zwischen den Kurven der Knotenspannungen und den gemessenen Kurven des Blutdrucks abnimmt.

7. Verfahren zum Messen von Strömungswiderständen von Blutgefäßen, wobei das Verfahren umfasst:
- Aussenden (301) elektromagnetischer Strahlung an die Blutgefäße, derart, dass die elektromagnetische Strahlung unterschiedliche Wellenlängen aufweist,
- Empfangen (302) eines Teils der von den Blutgefäßen reflektierten elektromagnetischen Strahlung,
- Erzeugen (303) eines Messsignals als Indikator für den empfangenen Teil der elektromagnetischen Strahlung, derart, dass das Messsignal wellenlängenspezifische Komponentensignale als Indikator für empfangenen, von den Blutgefäßen reflektierte Wellenlängen umfasst, wobei eine kürzere der Wellenlängen von kleineren Blutgefäßen reflektiert wird als eine längere der Wellenlängen,
- Erzeugen (304) von auf die Blutgefäße ausgeübtem mechanischem Druck,
- Finden (305) eines Abschnitts aus jedem der wellenlängenspezifischen Komponentensignale, dessen Hüllkurve sich im Verhältnis zur Zeit exponentiell ändert (~e^{αt}), wenn sich der mechanische Druck im Verhältnis zur Zeit linear ändert, und
- Erzeugen (306) einer Schätzung für einen Koeffizienten (α_{IR}, α_{R}, αγ, α_{G}, α_{B}) der Zeit für jedes der wellenlängenspezifischen Komponentensignale, der mit der exponentiellen Änderung in Zusammenhang steht, wobei der Zeitkoeffizient ein Indikator für die Nachgiebigkeit der Blutgefäße ist, die die betrachtete Wellenlänge reflektieren,
wobei das Verfahren das Optimieren (307) von Widerstandswerten eines Kreislaufmodells umfasst, um Differenzen zwischen Kurven von Knotenspannungen des Kreislaufmodells und gemessenen Kurven von Blutdrücken in den Blutgefäßen, die verschiedene der Wellenlängen reflektieren, zu minimieren, wobei Kondensatorwerte des Kreislaufmodells auf der Nachgiebigkeit der Blutgefäße basieren und die optimierten Widerstandswerte ein Indikator für die Strömungswiderstände der Blutgefäße sind.

8. Verfahren nach Anspruch 7, wobei die elektromagnetische Strahlung Wellenlängen aufweist, die aus den folgenden Bereichen ausgewählt sind: von 625 nm bis 1000 nm, von 565 nm bis 590 nm, von 500 nm bis 565 nm und von 450 nm bis 485 nm.

9. Verfahren nach Anspruch 7 oder 8, wobei das Verfahren das Umrechnen der wellenlängenspezifischen Komponentensignale in eine logarithmische Skala, das Finden eines Abschnitts in jedem umgerechneten wellenlängenspezifischen Komponentensignal, dessen Hüllkurve sich im Verhältnis zur Zeit linear ändert, und das Erzeugen einer Schätzung für eine Steigung (α_{IR}, α_{R}, α_{Y}, α_{G}, α_{B}) der Hüllkurve des umgerechneten wellenlängenspezifischen Komponentensignals im Verhältnis zu der linearen Änderung umfasst, wobei die Steigung der Zeitkoeffizient im Zusammenhang mit der exponentiellen Änderung des betrachteten wellenlängenspezifischen Komponentensignals ist.

10. Verfahren nach einem der Ansprüche 7 bis 9, wobei der mechanische Druck zu einer Fingerspitze oder einem Zeh geleitet wird.

11. Verfahren nach einem der Ansprüche 7 bis 10, wobei das Verfahren das Variieren der Widerstandswerte so lange, wie eine Summe der Zeitintegrale der Quadrate der Differenzen zwischen den Kurven der Knotenspannungen und den gemessenen Kurven des Blutdrucks abnimmt, umfasst.

12. Computerprogramm zum Messen von Strömungswiderständen von Blutgefäßen, wobei das Computerprogramm computerausführbare Anweisungen zum Steuern eines programmierbaren Verarbeitungssystems umfasst, um:
- einen Photoplethysmographiesensor zu steuern, um elektromagnetische Strahlung an die Blutgefäße auszusenden, einen Teil der von den Blutgefäßen reflektierten elektromagnetischen Strahlung zu empfangen und ein Messsignal als Indikator für den empfangenen Teil der elektromagnetischen Strahlung zu erzeugen, derart, dass die elektromagnetische Strahlung unterschiedliche Wellenlängen aufweist und das Messsignal wellenlängenspezifische Komponentensignale als Indikator für die empfangenen, von den Blutgefäßen reflektierten Wellenlängen umfasst, wobei eine kürzere der Wellenlängen von kleineren Blutgefäßen reflektiert wird als eine längere der Wellenlängen,
- ein Druckinstrument zu steuern, um einen mechanischen Druck zu erzeugen, der auf die Blutgefäße ausgeübt wird, und
- das programmierbare Verarbeitungssystem zu steuern, um in jedem der wellenlängenspezifischen Komponentensignale einen Abschnitt zu finden, dessen Hüllkurve sich im Verhältnis zur Zeit (t) exponentiell ändert (~e^{αYt}), wenn sich der mechanische Druck im Verhältnis zur Zeit linear ändert, und für jedes der wellenlängenspezifischen Komponentensignale eine Schätzung für einen Koeffizienten (α_{IR}, α_{R}, αγ, α_{G}, α_{B}) der Zeit zu erzeugen, der mit der exponentiellen Änderung in Zusammenhang steht, wobei der Zeitkoeffizient ein Indikator für die Nachgiebigkeit der Blutgefäße ist, die die betrachtete Wellenlänge reflektieren,
wobei das Computerprogramm ferner computerausführbare Anweisungen zum Steuern des programmierbaren Verarbeitungssystems umfasst, um Widerstandswerte eines Kreislaufmodells zu optimieren, um Differenzen zwischen Kurven von Knotenspannungen des Kreislaufmodells und gemessenen Kurven von Blutdrücken in den Blutgefäßen, die verschiedene der Wellenlängen reflektieren, zu minimieren, wobei Kondensatorwerte des Kreislaufmodells auf der Nachgiebigkeit der Blutgefäße basieren und die optimierten Widerstandswerte ein Indikator für die Strömungswiderstände der Blutgefäße sind.

13. Computerprogrammprodukt umfassend ein nichtflüchtiges computerlesbares Medium, das mit einem Computerprogramm gemäß Anspruch 12 codiert ist.

## Revendications

1. Appareil pour mesurer des résistances d'écoulement de vaisseaux sanguins, l'appareil comprenant :
- un capteur de photo-pléthysmographie (101, 201) configuré pour émettre un rayonnement électromagnétique vers les vaisseaux sanguins, pour recevoir une partie du rayonnement électromagnétique réfléchi par les vaisseaux sanguins, et pour produire un signal de mesure (122) indiquant la partie du rayonnement électromagnétique reçue, de façon à ce que le rayonnement électromagnétique ait différentes longueurs d'onde et le signal de mesure comprend des signaux de composante spécifique à la longueur d'onde (117-121) indiquant des longueurs d'ondes reçues réfléchies par les vaisseaux sanguins, où une plus courte des longueurs d'onde est réfléchie par de plus petits des vaisseaux sanguins qu'une plus longue des longueurs d'onde,
- un instrument de pression (102, 202) configuré pour produire une pression mécanique pouvant être commandée appliquée sur les vaisseaux sanguins, et
- un système de commande (103, 203) configuré pour trouver, à partir de chacun des signaux de composante spécifique à la longueur d'onde, une partie dont l'enveloppe présente un changement exponentiel (^{~}e^{αYt}) par rapport au temps (t) lorsque la pression mécanique change de manière linéaire par rapport au temps et produire, pour chacun des signaux de composante spécifique à la longueur d'onde, une estimation d'un coefficient (α_{IR}, α_{R}, α_{Y}, α_{G}, α_{B}) de temps lié au changement exponentiel, le coefficient de temps indiquant la compliance des vaisseaux sanguins réfléchissant la longueur d'onde en question,
dans lequel le système de commande est configuré pour optimiser des valeurs de résistance d'un modèle de circuit pour minimiser des différences entre des formes d'onde de tensions nodales du modèle de circuit et des formes d'onde mesurées des pressions sanguines étant dans les vaisseaux sanguins réfléchissant différentes longueurs d'onde, dans lequel des valeurs de condensateur du modèle de circuit sont basées sur les compliances des vaisseaux sanguins et les valeurs de résistance optimisées indiquent les résistances à l'écoulement des vaisseaux sanguins.

2. Appareil selon la revendication 1, dans lequel le capteur de photo-pléthysmographie (101) est configuré pour émettre, vers les vaisseaux sanguins, le rayonnement électromagnétique de façon à ce que le rayonnement électromagnétique ait les longueurs d'onde sélectionnées à partir des plages suivantes : de 625 nm à 1000 nm, de 565 nm à 590 nm, de 500 nm à 565 nm, et de 450 nm à 485 nm.

3. Appareil selon l'une quelconque revendication 1 ou 2, dans lequel le système de commande (103) est condensateur pour convertir les signaux de composante spécifique à la longueur d'onde à une échelle logarithmique, pour trouver à partir de chaque signal de composante spécifique à la longueur d'onde converti une partie dont l'enveloppe présente un changement linéaire par rapport au temps, et pour produire une estimation d'une pente (α_{IR}, α_{R}, α_{Y}, α_{G}, α_{B}) de l'enveloppe du signal de composante spécifique à la longueur d'onde converti lié au changement linéaire, la pente étant le coefficient de temps lié au changement exponentiel du signal de composante spécifique à la longueur d'onde en question.

4. Appareil selon l'une quelconque des revendications 1-3, dans lequel l'instrument de pression (102) comprend un générateur de force (104) et un élément de pression (105) configuré pour diriger la pression mécanique vers un bout du doigt ou un orteil selon un signal de commande généré par le système de commande (103).

5. Appareil selon l'une quelconque des revendications 1-3, dans lequel l'instrument de pression (202) comprend un brassard (241) et un système de pompe (242) configuré pour commander la pression de gaz à l'intérieur du brassard pour diriger la pression mécanique vers un bras.

6. Appareil selon l'une quelconque des revendications 1-5, dans lequel le système de commande (103) est configuré pour varier les valeurs de résistance jusqu'à aussi longtemps qu'une somme d'intégrales de temps de carrés de différences entre les formes d'onde des tensions nodales et les formes d'onde mesurées des pressions sanguines baisse.

7. Procédé de mesure de résistances à l'écoulement de vaisseaux sanguins, le procédé comprenant :
- d'émettre (301) un rayonnement électromagnétique vers les vaisseaux sanguins de façon à ce que le rayonnement électromagnétique présente différentes longueurs d'onde,
- de recevoir (302) une partie du rayonnement électromagnétique réfléchi par les vaisseaux sanguins,
- de produire (303) un signal de mesure indiquant la partie du rayonnement électromagnétique reçue de façon à ce que le signal de mesure comprenne des signaux de composante spécifique à la longueur d'onde indiquant des longueurs d'ondes reçues réfléchies par les vaisseaux sanguins, où une plus courte des longueurs d'onde est réfléchie par de plus petits des vaisseaux sanguins qu'une plus longue des longueurs d'onde,
- de produire (304) une pression mécanique pouvant être commandée appliquée sur les vaisseaux sanguins,
- de trouver (305), à partir de chacun des signaux de composante spécifique à la longueur d'onde, une partie dont l'enveloppe présente un changement exponentiel (^{~}e^{αYt}) par rapport au temps lorsque la pression mécanique change de manière linéaire par rapport au temps, et
- de produire (306), pour chacun des signaux de composante spécifique à la longueur d'onde, une estimation une estimation d'un coefficient (α_{IR}, α_{R}, α_{Y}, α_{G}, α_{B}) de temps lié au changement exponentiel, le coefficient de temps indiquant la compliance des vaisseaux sanguins réfléchissant la longueur d'onde en question,
dans lequel le procédé comprend d'optimiser (307) des valeurs de résistance d'un modèle de circuit pour minimiser des différences entre des formes d'onde de tensions nodales du modèle de circuit et des formes d'onde mesurées des pressions sanguines étant dans les vaisseaux sanguins réfléchissant différentes longueurs d'onde, dans lequel des valeurs de condensateur du modèle de circuit sont basées sur les compliances des vaisseaux sanguins et les valeurs de résistance optimisées indiquent les résistances à l'écoulement des vaisseaux sanguins.

8. Procédé selon la revendication 7, dans lequel le rayonnement électromagnétique présente des longueurs d'onde sélectionnées à partir des plages suivantes : de 625 nm à 1000 nm, de 565 nm à 590 nm, de 500 nm à 565 nm, et de 450 nm à 485 nm.

9. Procédé selon l'une quelconque revendication 7 ou 8, dans lequel le procédé comprend de convertir les signaux de composante spécifique à la longueur d'onde à une échelle logarithmique, de trouver à partir de chaque signal de composante spécifique à la longueur d'onde converti une partie dont l'enveloppe présente un changement linéaire par rapport au temps, et de produire une estimation d'une pente (α_{IR}, α_{R}, α_{Y}, α_{G}, α_{B}) de l'enveloppe du signal de composante spécifique à la longueur d'onde converti lié au changement linéaire, la pente étant le coefficient de temps lié au changement exponentiel du signal de composante spécifique à la longueur d'onde en question.

10. Procédé selon l'une quelconque des revendications 7-9, dans lequel la pression mécanique est dirigée vers un bout du doigt ou un orteil.

11. Procédé selon l'une quelconque des revendications 7-10, dans lequel le procédé comprend de faire varier les valeurs de résistance jusqu'à aussi longtemps qu'une somme d'intégrales de temps de carrés de différences entre les formes d'onde des tensions nodales et les formes d'onde mesurées des pressions sanguines baisse.

12. Programme informatique pour mesurer des résistances d'écoulement de vaisseaux sanguins, le programme informatique comprenant d'exécuter des instructions exécutables pour commander un système de traitement programmable à :
- commander un capteur de photo-pléthysmographie à émettre un rayonnement électromagnétique vers les vaisseaux sanguins, à recevoir une partie du rayonnement électromagnétique réfléchi par les vaisseaux sanguins, et à produire un signal de mesure indiquant la partie du rayonnement électromagnétique reçue, de façon à ce que le rayonnement électromagnétique ait différentes longueurs d'onde et le signal de mesure comprend des signaux de composante spécifique à la longueur d'onde indiquant des longueurs d'ondes reçues réfléchies par les vaisseaux sanguins, où une plus courte des longueurs d'onde est réfléchie par de plus petits des vaisseaux sanguins qu'une plus longue des longueurs d'onde,
- commander un instrument de pression pour produire une pression mécanique pouvant être commandée appliquée sur les vaisseaux sanguins, et
- à commander le système de commande programmable pour trouver, à partir de chacun des signaux de composante spécifique à la longueur d'onde, une partie dont l'enveloppe présente un changement exponentiel (^{~}e^{αYt}) par rapport au temps (t) lorsque la pression mécanique change de manière linéaire par rapport au temps et produire, pour chacun des signaux de composante spécifique à la longueur d'onde, une estimation d'un coefficient (α_{IR}, α_{R}, α_{Y}, α_{G}, α_{B}) de temps lié au changement exponentiel, le coefficient de temps indiquant la compliance des vaisseaux sanguins réfléchissant la longueur d'onde en question,
dans lequel le programme informatique comprend en outre des instructions exécutables sur ordinateur pour commander le système de traitement programmable à optimiser des valeurs de résistance d'un modèle de circuit pour minimiser des différences entre des formes d'onde de tensions nodales du modèle de circuit et des formes d'onde mesurées des pressions sanguines étant dans les vaisseaux sanguins réfléchissant différentes longueurs d'onde, dans lequel des valeurs de condensateur du modèle de circuit sont basées sur les compliances des vaisseaux sanguins et les valeurs de résistance optimisées indiquent les résistances à l'écoulement des vaisseaux sanguins.

13. Produit de programme informatique comprenant un support non-transitoire lisible sur ordinateur encodé avec un programme informatique selon la revendication 12.
